# EUROPEAN PATENT APPLICATION

(11) **EP 2 256 513 A2**
(43) Date of publication of application: **01.12.2010**
(21) Application number: 10159835.7
(22) Date of filing: 14.04.2010
(51) Int. Cl.: G01S 7/52

(54) **Apparatus and method for embodying elastic image**

(30) Priority: 14.05.2009 KR 20090042088
(71) Applicant: Medison Co., Ltd., Kangwon-do 250-875 (KR)
(72) Inventor: Shin, Dong Kuk, Seoul 139-224 (KR); Kim, Jong Sik, Seoul 143-757 (KR)
(74) Representative: Schmid, Wolfgang

(57) **Abstract**

Disclosed is an elastic image embodying apparatus including an interpolation unit to perform interpolation of a maximum pressure and a minimum pressure of ultrasound image data to generate first data, an image generating unit to generate a pyramid image by using the generated first data, a map generating unit to generate a motion map by using the generated pyramid image, a displacement calculating unit to calculate a displacement based on the generated motion map, and an image embodying unit to embody an elastic image by using the calculated displacement.

## Description

### APPARATUS AND METHOD FOR EMBODYING ELASTIC IMAGE

### CROSS-REFERENCE TO RELATED APPLICATION

This application claims the benefit of Korean Patent Application No. 10-2009-0042088, filed on May 14, 2009, in the Korean Intellectual Property Office, the disclosure of which is incorporated herein by reference.

### BACKGROUND

### 1. Field of the Invention

The present invention relates to an elastic image embodying apparatus and method.

### 2. Description of the Related Art

Most medical images created using ultrasound are Brightness (B)-mode images. The B-mode image, which visualizes a sound impedance of a medium, has difficulty in visualizing a cancer, a tumor, and the like existing in a soft tissue since a boundary is not clearly distinguished, thereby having difficulty in diagnosing a lesion. Accordingly, various researches have been conducted, such as an attenuation coefficient, a non-linear parameter (B/A), a sound velocity distribution, a modulus of an elasticity image, and the like, as methods of calculating and visualizing a characteristic value of a tissue. However, these methods do not produce a good result due to a complex interaction between the ultrasound and a human body. An elastography of the above researches relatively easily measures the tissue since a difference in characteristic between tissues is sufficient, and thus, various researches related to the elastography have been conducted.

The elastography visualizes the modulus of the elasticity of the tissue, and uses a fact that an elasticity of the tissue relates to a pathological condition. Actually, a tissue of the cancer or the tumor has a characteristic of being harder than a general soft tissue. When an outside force is given, the cancer or the tumor has an insignificant change since an elasticity of the tissue is high, whereas the soft tissue has a greater change than the cancer tissue. The elastography performs visualization based on the described characteristic, and provides information about a degree of a solidity of the tissue, which is not provided by an existing B-mode image method, and thus contributes a significant breakthrough in diagnosing a cancer. The elastography is a field drawing a large amount of attention.

The elastography may be applicable to a field that visualizes a characteristic of a tissue, such as detection and classification of breast cancer and prostate cancer, a skin biopsy, monitoring of a kidney transplant, monitoring of cancer treatment using high intensity focused ultrasound (HIFU), and the like.

### SUMMARY

An aspect of the present invention provides an elastic image embodying apparatus and method that may calculate a displacement based on a three-dimensional (3D) motion direction of an image, thereby increasing a quality of an elastic image

Another aspect of the present invention also provides an elastic image embodying apparatus and method that may calculate a 3D motion direction of an image by using a plurality of sequential data, thereby minimizing an amount of calculation and providing a fast process rate.

According to an aspect of the present invention, there is provided an elastic image embodying apparatus, the apparatus including an interpolation unit to perform interpolation of a maximum pressure and a minimum pressure of ultrasound image data to generate first data, an image generating unit to generate a pyramid image by using the generated first data, a map generating unit to generate a motion map by using the generated pyramid image, a displacement calculating unit to calculate a displacement based on the generated motion map, and an image embodying unit to embody an elastic image by using the calculated displacement.

According to an aspect of the present invention, there is provided an elastic image embodying method, the method including performing interpolation of a maximum pressure and a minimum pressure of ultrasound image data and generating first data, generating a pyramid image by using the generated first data, generating a motion map by using the generated pyramid image, calculating a displacement based on the generated motion map, and embodying an elastic image by using the calculated displacement.

Additional aspects, features, and/or advantages of the invention will be set forth in part in the description which follows and, in part, will be apparent from the description, or may be learned by practice of the invention.

### EFFECT

According to embodiments of the present invention, a displacement is calculated based on a 3D motion direction of an image, thereby increasing a quality of an elastic image.

According to embodiments of the present invention, a 3D motion direction is calculated by using a plurality of sequential data, thereby minimizing an amount of calculation and increasing a process rate.

According to embodiments of the present invention, a direction is calculated from an entire space and a one-dimensional (1D) direction is extracted from a 3D space, and thus, a displacement is calculated based on a 3D direction while performing a correlation process based on the 1D direction Accordingly, even when a two-dimensional (2D) elastic image is embodied, an image quality increases by decreasing a distortion of a motion, and also a 3D elastic image may be efficiently embodied in real time.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a diagram illustrating a point in time where a three-dimensional (3D) probe obtains data and data configuration for interpolation;
FIG. 2 is a block diagram illustrating an elastic image embodying apparatus according to embodiments of the present invention;
FIG. 3 is a diagram illustrating an example of a pyramid image generated according to embodiments of the present invention;
FIGS. 4A and 4B are diagrams illustrating an example of a motion map generated according to embodiments of the present invention;
FIG. 5 is a diagram illustrating an example of a space where motion information is extracted according to embodiments of the present invention;
FIG. 6 is a diagram illustrating an example of calculating a displacement by using a correlation scheme; and
FIG. 7 is a flowchart illustrating an elastic image embodying method according to embodiments of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

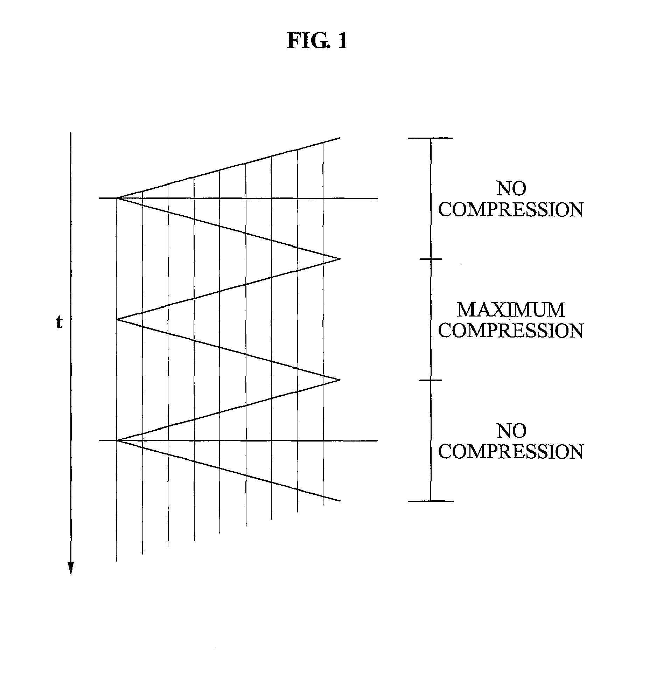

FIG. 1 is a diagram illustrating a point in time where a three-dimensional (3D) probe obtains data and data configuration for interpolation.

3D data may be obtained by moving a 3D mechanical probe or a multi-dimension electronic array probe up and down in the same manner of a freehand scheme. However, the 3D mechanical probe may obtain data by oscillating right and left, and thus the point in time where data is obtained is different as illustrated in FIG. 1.

In FIG. 1, a zigzag line represents a point in time where the 3D mechanical probe obtains data. Also, the point in time where the 3D mechanical probe obtains the data is late due to a configuration of the 3D mechanical probe, and thus, to correct the data being obtained late, the 3D mechanical probe may use interpolation and may obtain data of a relatively narrow time interval. This may prevent aliasing that may occur when one-dimensional (1D) data is obtained based on a direction of 3D data.

Also, in FIG. 1, a vertical line represents a plurality of data. Based on the configuration of FIG.1, an interpolation of data may be performed. In this instance, a precondition is that a single motion from left->right or right-> left is a minimum compression and next motion is maximum compression, and thus, one full left->right->left or right->left->right motion is represented by the minimum compression and the maximum compression.

This method may enable the probe to move vertically at every reciprocating sweep in the same manner that the mechanical-swept 3D probe moves right and left, and also, may only provide a pressure measurement when using a pressure gauge and the probe is fixed.

The pressure gauge may be contained outside the probe or inside an ultrasound image diagnostic system, and may control a degree of the pressure. The pressure gauge may select one of various predetermined levels as the degree of the pressure. Accordingly, the degree of the pressure may be determined based on a function of controlling the degree of the pressure by selecting one of the predetermined levels, and thus, the degree of the pressure is determined without a sensor, thereby quantifying an elasticity of ultrasound image data.

Also, the method may be applicable to the freehand scheme, and in this instance, the elasticity of ultrasound image data may not be quantified since the pressure is not constant. However, the freehand scheme may obtain the ultrasound image data by repeatedly providing and removing pressure at every reciprocating swept, and an elasticity of the ultrasound image data may be expressed by a relative normalization value for each single period of interpolation, since the pressure may be different every time that the pressure is provide.

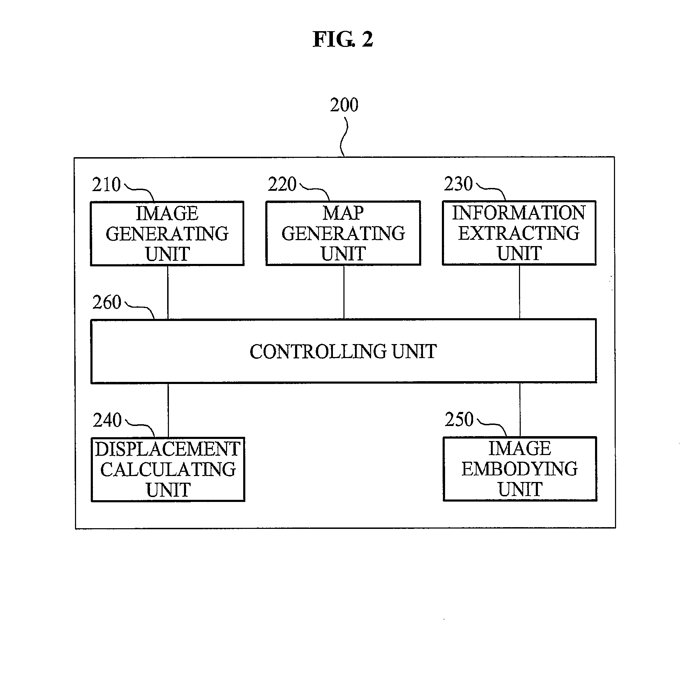

FIG. 2 is a block diagram illustrating an elastic image embodying apparatus according to embodiments of the present invention. Here, the elastic image embodying apparatus may be contained and embodied in an ultrasound image diagnostic system.

Referring to FIG. 2, the elastic image embodying apparatus 200 may include an image generating unit 210, a map generating unit 220, an information extracting unit 230, a displacement calculating unit 240, an image embodying unit 250, and controlling unit 260.

The image generating unit 210 may generate a pyramid image by using first data. Here, the first data includes In-phase and Quadrature-phase (IQ) data including at least three sequential frames or radio frequency (RF) data

As a number of the sequential frames increases, an accuracy of a motion map (3D motion direction map) generated by the map generating unit 220 increases. Accordingly, the image generating unit 210 may determine the number of sequential frames based on a relationship between a process rate of the elastic image embodying apparatus 200 and the accuracy of the motion map.

In other words, the image generating unit 210 may generate the pyramid image to have a multi-level structure, and may determine a depth of a multi-level based on at least one of the process rate of the elastic image embodying apparatus and a resolution of a motion direction of the first data.

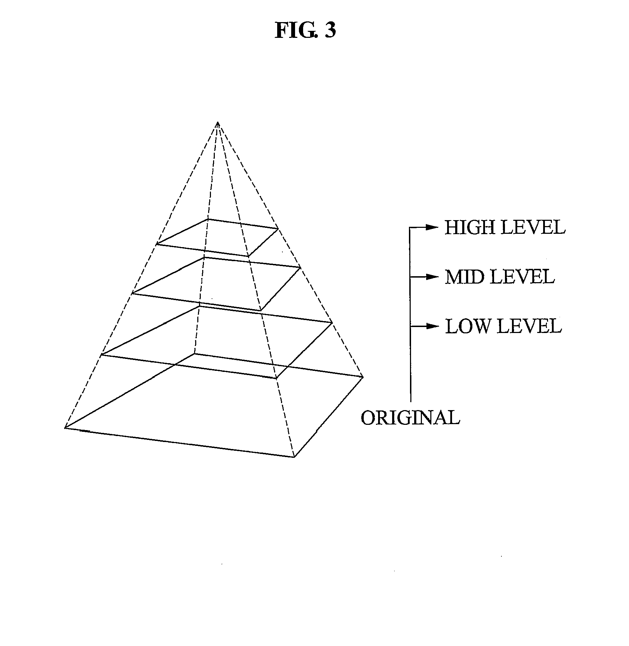

As an example, when the depth of the multi-level is determined to be three, the image generating unit 210 may generate the pyramid image by using the at least three sequential frames (high level, mid level, and low level frames) as illustrated in FIG. 3. For reference, FIG. 3 is a diagram illustrating an example of a pyramid image generated according to embodiments of the present invention

In this matter, the image generating unit 210 may generate the pyramid image by using an appropriate number of first data, and thereby preparing a condition to decrease an amount of calculation while the map generating unit 220 generates the motion map.

The map generating unit 220 may generate the motion map by using the generated pyramid image. Here, the motion map used for searching for a direction of a 3D motion, is motion information based on motions of basic information (edge) in the first data. In other words, the motion map is a concept including a degree of motion (motion value), a motion direction, a motion speed, and the like.

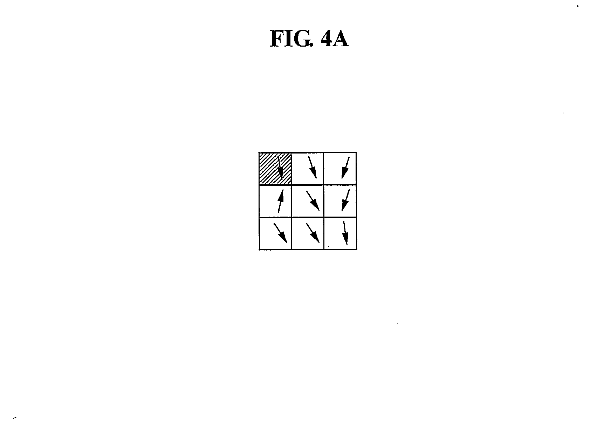

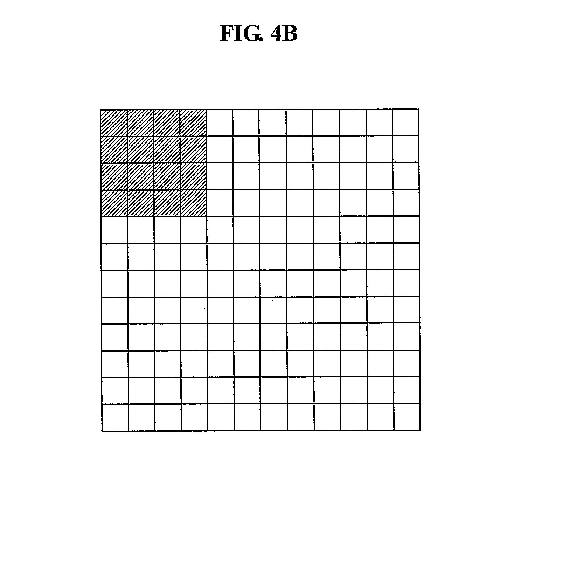

As an example, the motion map may include a direction of a motion as illustrated in FIG. 4A, and FIG. 4B represents a magnified FIG. 4A. The motion map of FIGS. 4A and 4B are examples of a 2D motion map, and a 3D motion map may be embodied by extending the 2D motion map to a temporal axis. For reference, FIG. 4A and 4B are diagrams illustrating an example of a motion map generated according to embodiments of the present invention.

Particularly, the map generating unit 220 may calculate a motion direction of at least one of an X (horizontal)-axis, a Y (vertical)-axis, and a Z (temporal)-axis with respect to the first data by using the generated pyramid image, and may generate the motion map based on the calculated motion direction.

As an example, it is assumed that the generated pyramid image includes an 'image A', an 'image B', and an 'image C'. In this instance, the map generating unit 220 may calculate a motion direction of at least one of a horizontal axis, a vertical axis, and a temporal axis for each of the 'image A', the 'image B', and the 'image C' by using a block matching scheme or a correlation scheme. Also, the map generating unit 220 may generate the motion map by using the calculated motion directions.

In this manner, the map generating unit 220 may generate the motion map including some information such as a motion direction and the like. Accordingly, the map generating unit 220 may minimize an amount of calculation to generate the motion map.

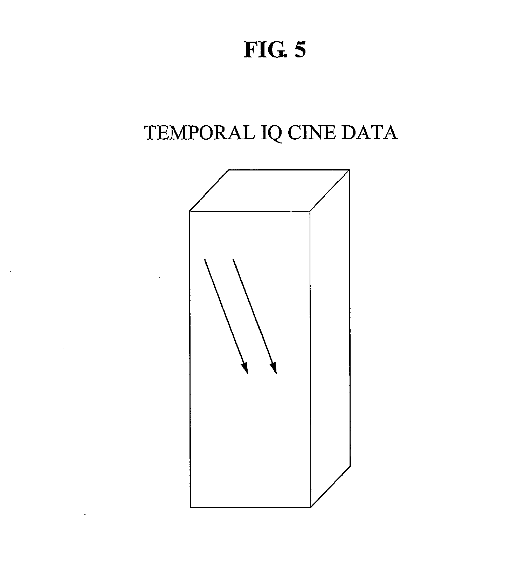

The information extracting unit 230 may extract motion information from second data by using the generated motion map. Here, the second data may include a frame (temporal IQ input cine data) that is inputted after the first data. As an example, the second data may be embodied as illustrated in FIG. 5. For reference, FIG. 5 is a diagram illustrating an example of a space where motion information is extracted according to embodiments of the present invention.

Particularly, the information extracting unit 230 may predict at least one of a motion direction of the second data and a maximum motion value (a degree of maximum motion) of the second data by using the generated motion map. Also, the information extracting unit 230 may extract the motion information from the second data based on at least one of the motion direction of the second data and the maximum motion value of the second data.

The displacement calculating unit 240 may calculate a displacement by using the extracted motion information. Here, the displacement may include a 3D displacement. The displacement calculating unit 240 may calculate the displacement by applying the extracted motion information to a cross/auto correlation scheme, and the like.

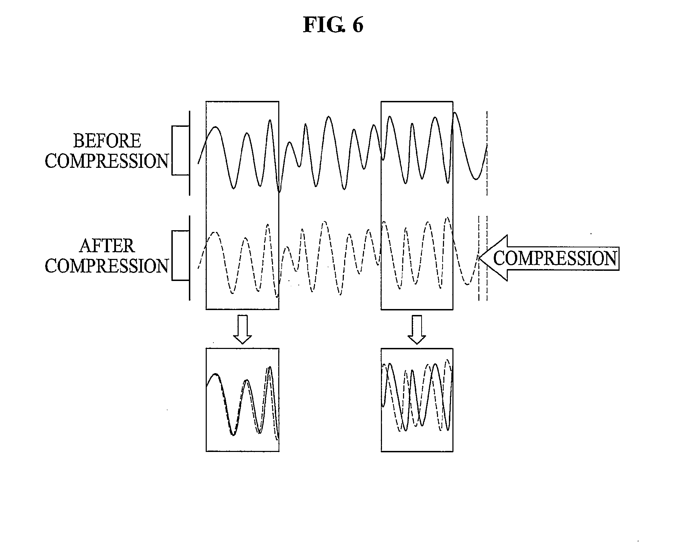

As an example, as illustrated in FIG. 6, a signal before compression is an ultrasound signal before a surface of a medium (human body) is compressed, namely, a basic signal, and a signal after the compression is an ultrasound signal after the surface of the medium is compressed. The displacement calculating unit 240 may measure a correlation between the ultrasound signals before and after the compression, may calculate a movement between the signals before and after the compression based on the measured correlation to calculate the displacement of the medium. Here, the compression may be performed by a pressure gauge contained outside a probe or inside an ultrasound image diagnostic system. For reference, FIG. 6 is a diagram illustrating an example of calculating a displacement by using a correlation scheme.

The image embodying unit 250 may embody an elastic image by using the calculated displacement. That is, the image embodying unit 250 may embody the elastic image by mapping a corresponding color to an image based on a degree of the calculated displacement. In this instance, the image embodying unit 250 may process the elastic image by using a post processing, and thereby increasing a quality of the elastic image.

The controlling unit 260 may generally control the elastic image embodying apparatus 200, namely, the image generating unit 210, the map generating unit 220, the information extracting unit 230, the displacement calculating unit 240, the image embodying unit 250, and the like.

In addition, although not illustrated, the elastic image embodying apparatus 200 may further include an interpolation unit. The interpolation unit may perform interpolation of a maximum pressure of ultrasound image data and a minimum pressure of the ultrasound image data to generate the first data. Accordingly, the image generating unit 210 may generate the pyramid image by using the generated first data.

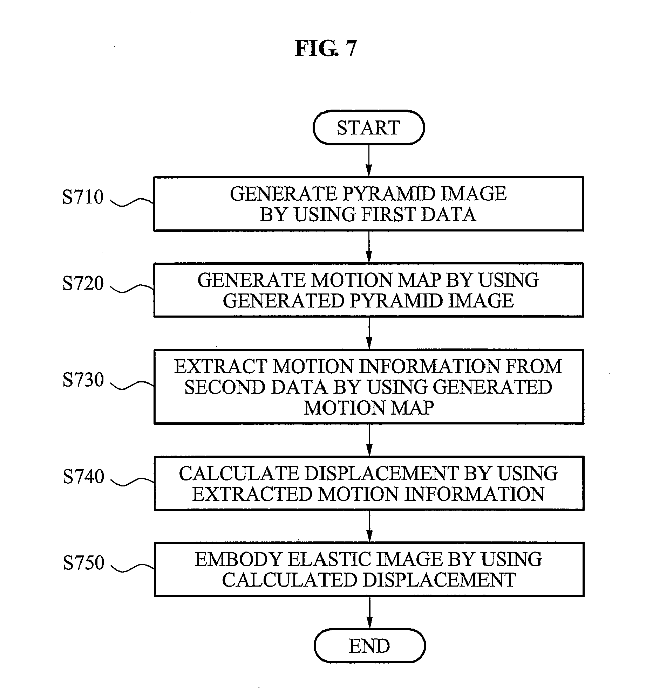

FIG. 7 is a flowchart illustrating an elastic image embodying method according to embodiments of the present invention. Here, the elastic image embodying method may be performed by the elastic image embodying apparatus of FIG. 2.

Referring to FIG. 7, first, the elastic image embodying apparatus may perform interpolation of a maximum pressure of ultrasound image data and a minimum pressure of the ultrasound image data, and may generate the first data.

Next, in operation S710, the elastic image embodying apparatus generates a pyramid image by using the generated first data.

In this instance, the elastic image embodying apparatus may generate the pyramid image to have a multi-level structure, and may determine a depth of a multi-level based on at least one of a process rate of the elastic image embodying apparatus and a resolution of a motion direction of the first data.

Next, in operation S720, the elastic image embodying apparatus generates a motion map by using the generated pyramid image.

That is, the elastic image embodying apparatus may calculate a motion direction of at least one of an X (horizontal)-axis, a Y (vertical)-axis, and a Z (temporal)-axis with respect to the first data by using the generated pyramid image, and may generate the motion map based on the calculated motion direction.

Next, in operation S730, the elastic image embodying apparatus extracts motion information from second data by using the generated motion map.

That is, the elastic image embodying apparatus may predict at least one of a motion direction of the second data and a maximum motion value (a degree of maximum motion) of the second data by using the generated motion map. Next, the elastic image embodying apparatus may extract the motion information from the second data based on at least one of the motion direction of the second data and the maximum motion value of the second data.

Next, in operation S740, the elastic image embodying apparatus calculates a displacement by using the extracted motion information. In this instance, the elastic image embodying apparatus may calculate the displacement by applying the extracted motion information to a cross/auto correlation scheme and the like.

Next, in operation S750, the elastic image embodying apparatus embodies an elastic image by using the calculated displacement. That is, the elastic image embodying apparatus may embody the elastic image by mapping a corresponding color to an image according to a degree of the calculated displacement.

The method according to the above-described exemplary embodiments of the present invention may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defmed by the claims and their equivalents.

## Claims

1. An elastic image embodying apparatus, the apparatus comprising:
an interpolation unit to perform interpolation of a maximum pressure and a minimum pressure of ultrasound image data to generate first data;
an image generating unit to generate a pyramid image by using the generated first data;
a map generating unit to generate a motion map by using the generated pyramid image;
a displacement calculating unit to calculate a displacement based on the generated motion map; and
an image embodying unit to embody an elastic image by using the calculated displacement.

2. The apparatus of claim 1, further comprising:
an information extracting unit to extract motion information from second data that is different from the first data, by using the generated motion map,
wherein the displacement calculating unit calculates the displacement by using the extracted motion information.

3. The apparatus of claim 2, wherein the information extracting unit predicts at least one of a motion direction of the second data or a maximum motion value of the second data by using the generated motion map, and extracts the motion information from the second data based on at least one of the motion direction and the maximum motion value.

4. The apparatus of claim 1, wherein the map generating unit calculates, by using the generated pyramid image, a motion direction of at least one of an X-axis, a Y-axis, or a Z-axis with respect to the first data, and generates the motion map based on the calculated motion direction.

5. The apparatus of claim 1, wherein the image generating unit generates the pyramid image to have a multi-level structure, and determines a depth of a multi-level based on at least one of a process rate of the elastic image embodying apparatus and a resolution of the motion direction of the first data.

6. The apparatus of claim 1, wherein the first data includes at least three sequential frames.

7. An ultrasound image diagnostic system including the apparatus of claim 1.

8. An elastic image embodying method, the method comprising:
performing interpolation of a maximum pressure and a minimum pressure of ultrasound image data and generating first data;
generating a pyramid image by using the generated first data;
generating a motion map by using the generated pyramid image;
calculating a displacement based on the generated motion map; and
embodying an elastic image by using the calculated displacement.

9. The method of claim 8, further comprising:
extracting motion information from second data different from the first data by using the generated motion map,
wherein the calculating of the displacement calculates the displacement by using the extracted motion information.

10. The method of claim 9, wherein the extracting of the motion information comprises:
predicting at least one of a motion direction of the second data or a maximum motion value of the second data, by using the generated motion map; and
extracting the motion information from the second data based on at least one of the motion direction and the maximum motion value.

11. The method of claim 8, wherein the generating of the motion map comprises:
calculating a motion direction of at least one of an X-axis, a Y-axis, and a Z-axis with respect to the first data; and
generating the motion map based on the calculated motion direction.

12. The method of claim 8, wherein the generating of the pyramid image comprises:
generating the pyramid image to have a multi-level structure; and
determining a depth of the multi-level based on at least one of a process rate of the elastic image embodying apparatus and a resolution of the motion direction of the first data.

13. At least one medium comprising computer readable instructions implementing the method of claim 8.
